# EUROPEAN PATENT APPLICATION

(11) **EP 2 660 605 A1**
(43) Date of publication of application: **06.11.2013**
(21) Application number: 11853259.7
(22) Date of filing: 09.11.2011
(51) Int. Cl.: G01N 33/66, H04M 1/00

(54) **PORTABLE COMMUNICATION DEVICE, CONTROL METHOD, AND VOICE OUTPUT SYSTEM**

(30) Priority: 28.12.2010 JP 2010293748
(71) Applicant: Terumo Kabushiki Kaisha, Tokyo 151-0072 (JP)
(72) Inventor: TAKAHASHI, Masayuki, Ashigarakami-gun Kanagawa 259-0151 (JP)
(74) Representative: TBK
(86) International application number: PCT/JP2011/006261
(87) International publication number: WO 2012/090374

(57) **Abstract**

This invention implements a voice output function while maintaining the operability and portability of a blood sugar measurement device. This invention provides a portable phone (300) including an application for implementing the voice output function. The portable phone includes a communication unit (310) that starts communication to receive a signal transmitted from the blood sugar measurement device by activating the application, a voice data storage unit (373) that stores voice data representing the operation contents of the blood sugar measurement device, a voice selection unit (375) that, upon receiving a signal including an identifier corresponding to the internal state of the blood sugar measurement device, selects the voice data according to the internal state out of the voice data stored in the voice data storage unit, and a reproduction processing unit (374) that outputs the voice data selected by the voice selection unit (375) .

## Description

### TECHNICAL FIELD

The present invention relates to a portable communication device, a method of controlling the same, and a voice output system including the portable communication device and a medical device.

### BACKGROUND ART

Conventionally, devices having a voice output function of notifying, by voice output, a user of an internal state at the time of measurement or a measurement result and the like upon completing measurement have found widespread use in the field of medical devices.

For example, a blood sugar measurement device can output, as a voice, operation guidance corresponding to the transition of the internal state at the time of measurement or blood sugar level data that is a measurement result. The subject of the blood sugar measurement device is often a diabetic and visually disordered. Hence, the voice output function is very convenient for the subject.

### CITATION LIST

### PATENT LITERATURE

PTL 1: Japanese Patent No. 4395146

### SUMMARY OF INVENTION

### TECHNICAL PROBLEM

However, the device having the voice output function has large outer dimensions and therefore has poor operability and portability.

The present invention has been made in consideration of the above-described problem.

### SOLUTION TO PROBLEM

A portable communication device according to the present invention has the following arrangement. That is, a portable communication device including an application for implementing a voice output function comprises communication means for starting communication to receive a signal transmitted from a predetermined medical device by activating the application, storage means for storing voice data representing operation contents of the predetermined medical device, selection means for, when a signal including an identifier corresponding to an internal state of the predetermined medical device is received from the predetermined medical device, selecting the voice data according to the internal state out of the voice data stored in the storage means, and output means for outputting the voice data selected by the selection means.

### ADVANTAGEOUS EFFECTS OF INVENTION

According to the present invention, it is possible to implement a voice output function while maintaining the operability and portability of a medical device.

Other features and advantages of the present invention will be apparent from the following descriptions taken in conjunction with the accompanying drawings, in which like reference characters designate the same or similar parts throughout the figures thereof.

### BRIEF DESCRIPTION OF DRAWINGS

The accompanying drawings, which are incorporated in and constitute a part of the specification, illustrate embodiments of the invention and, together with the description, serve to explain the principles of the invention.
Fig. 1 is a view showing the outer arrangement of a blood sugar measurement system 100 including a portable communication device according to an embodiment of the present invention;
Fig. 2 is a block diagram showing the functional arrangement of a measurement device main body 210 of a blood sugar measurement device 200;
Fig. 3 is a block diagram showing the functional arrangement of a portable communication device 300 and that of an voice output control program;
Fig. 4A is a flowchart showing the procedure of blood sugar measurement/output processing of the blood sugar measurement system 100;
Fig. 4B is a flowchart showing the procedure of blood sugar measurement/output processing of the blood sugar measurement system 100;
Fig. 5 is a flowchart showing the procedure of reproduction processing of the portable phone 300;
Fig. 6A is a flowchart showing the procedure of history call/output processing of the blood sugar measurement system 100; and
Fig. 6B is a flowchart showing the procedure of history call/output processing of the blood sugar measurement system 100.

### DESCRIPTION OF EMBODIMENTS

The embodiments of the present invention will now be described in detail with reference to the accompanying drawings as need. Note that the present invention is not limited to the following embodiments, and can be changed appropriately.

A blood sugar measurement system (voice output system) will be exemplified below, which uses a blood sugar measurement device as a medical device and a so-called portable phone as a portable communication device including an application for implementing the voice output function. However, the present invention is not limited to this.

### [First Embodiment]

### <1. Outer Arrangement of Blood Sugar Measurement System>

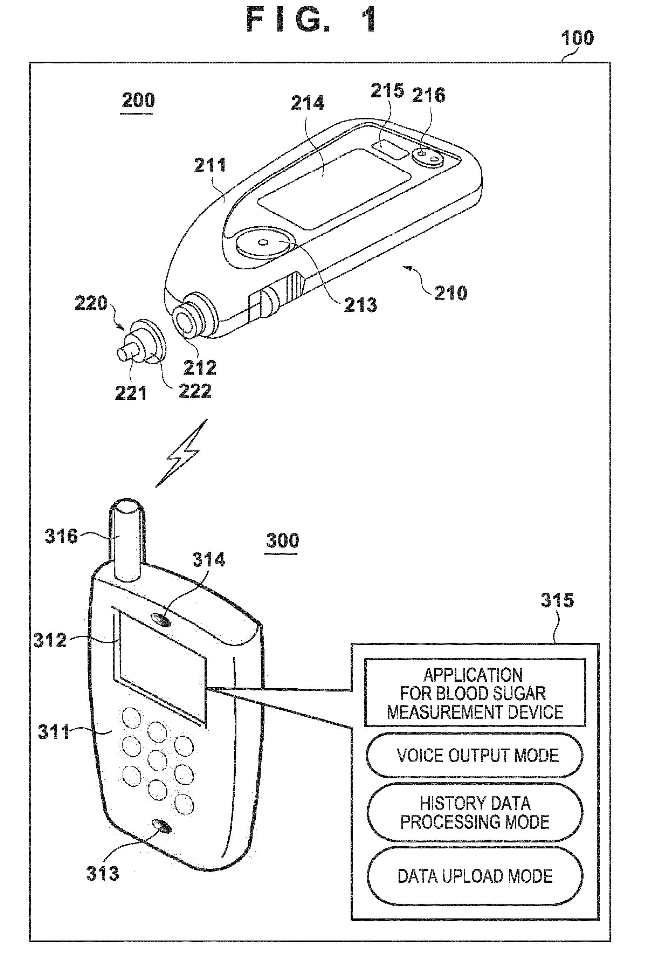

Fig. 1 is a view showing the outer arrangement of a blood sugar measurement system 100 serving as a voice output system including a portable phone 300 according to an embodiment of the present invention. As shown in Fig. 1, the blood sugar measurement system 100 includes a blood sugar measurement device 200 that measures a blood sugar level, and the portable phone 300 that provides voice guidance on the operation contents of the blood sugar measurement device 200 at the time of measurement or outputs measurement data (blood sugar level data) measured by the blood sugar measurement device 200 as a voice. The blood sugar measurement device 200 and the portable phone 300 are connected so as to be communicable with each other. Note that the communication between the blood sugar measurement device 200 and the portable phone 300 can be either wired or wireless. Wireless communication can be communication using a radio wave or communication using light or a sound wave.

The blood sugar measurement device 200 includes a measurement device main body 210 and a measurement chip 220 attached to the measurement device main body 210.

The measurement chip 220 holds a blood specimen. The measurement chip 220 includes a holder 222 provided with a tubular portion 221. Test paper (not shown) is fixed in the holder 222.

The tubular portion 221 guides a blood specimen from the opening portion at the distal end into the holder 222 by capillarity. The blood specimen guided through the tubular portion 221 is absorbed by the test paper in the holder 222. The test paper is impregnated with a coloring reagent that exhibits a color reaction upon reacting with glucose.

The measurement device main body 210 calculates the blood sugar level of the blood specimen held by the measurement chip 220. More specifically, the test paper that has absorbed the blood specimen is irradiated with two light components having different wavelengths, and the intensity of each reflected light is measured to calculate the blood sugar level.

A measurement unit 212 that irradiates the test paper with two light components having different wavelengths and measures the intensity of each reflected light is arranged at the distal end of a housing 211 of the measurement device main body 210. In addition, a control unit (not shown) that controls the measurement unit 212 and executes various kinds of operations for photometric data acquired by the measurement unit 212 is arranged in the housing 211.

The housing 211 further includes, on its surface, a power switch 213 used to input an on/off instruction to a power supply for supplying power to the blood sugar measurement device 200, a display unit 214 that displays blood sugar level data calculated by the control unit or an internal state detected by the control unit, a history call button 215 used to call past blood sugar level data calculated by the control unit and stored in association with a measurement date/time and displays the data on the display unit 214, and a reproduction button 216 used to re-output contents output by the portable phone 300 as a voice.

The portable phone 300 includes an antenna 316, an operation unit 311 including a ten-key pad and the like, a voice output unit 314, and a voice input unit 313. The portable phone 300 is configured to be able to perform speech communication, mail exchange, or transmission/reception of various kinds of data with another portable phone or the like.

In addition, an application for the blood sugar measurement device is installed in the portable phone 300 according to this embodiment. The application has a function of outputting a predetermined voice in accordance with an instruction from the blood sugar measurement device 200 or processing blood sugar level data transmitted from the blood sugar measurement device 200 and uploading it to a predetermined data server.

Reference numeral 315 indicates an example of an operation screen displayed on the display unit 312 when the application for the blood sugar measurement device is activated. As indicated by the operation screen 315, the application for the blood sugar measurement device has a voice output mode for outputting a predetermined voice in accordance with an instruction from the blood sugar measurement device 200 at the time of measurement, a history data processing mode for displaying past blood sugar level data transmitted from the blood sugar measurement device 200 and stored in the portable phone 300 in association with a reception date/time or applying predetermined statistical processing to the blood sugar level data, and a data upload mode for uploading blood sugar level data that is a measurement result transmitted from the blood sugar measurement device 200 to a predetermined data server (for example, a data server in a hospital).

Note that in the portable phone 300 according to this embodiment, processing (voice output control processing) in the voice output mode out of the above-described modes of the application for the blood sugar measurement device will mainly be described below.

### <2. Functional Arrangement of Blood Sugar Measurement Device>

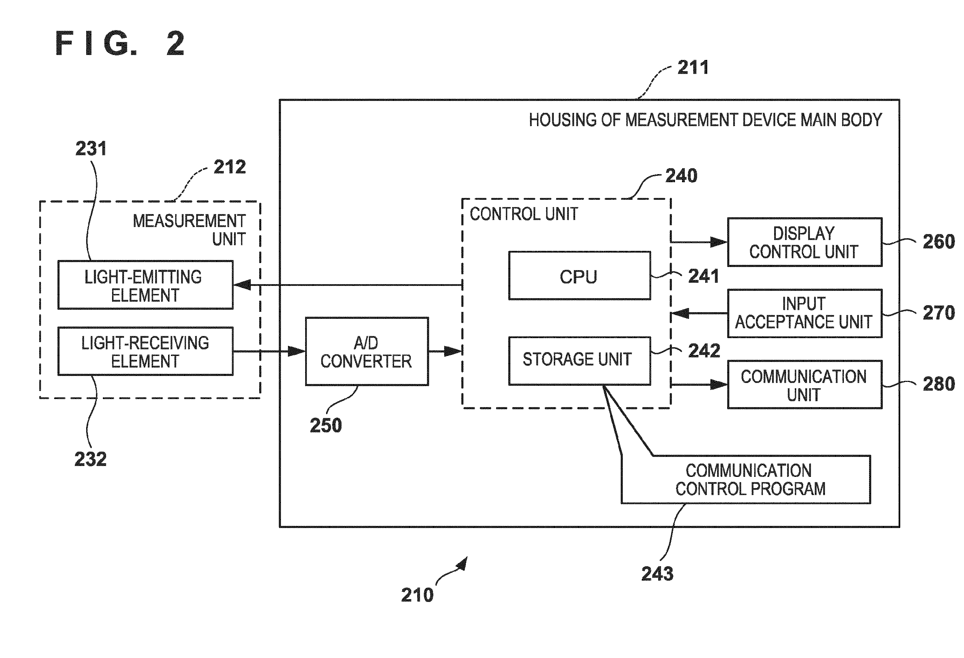

The functional arrangement of the measurement device main body 210 of the blood sugar measurement device 200 will be described next. Fig. 2 is a block diagram showing the functional arrangement of the measurement device main body 210 of the blood sugar measurement device 200.

As shown in Fig. 2, the measurement unit 212 of the measurement device main body 210 is provided with a light-emitting element 231 and a light-receiving element 232. The light-emitting element 231 emits light based on a light emission instruction from a control unit 240. The light-receiving element 232 receives reflected light of light emitted by the light-emitting element 231 to irradiate the test paper that has absorbed a blood specimen, thereby generating photometric data.

On the other hand, the control unit 240, an A/D converter 250, a display control unit 260, an input acceptance unit 270, and a communication unit 280 are arranged in the housing 211 of the measurement device main body 210.

The A/D converter 250 A/D-converts the photometric data generated by the light-receiving element 232 and inputs it to the control unit 240.

The control unit 240 includes a CPU 241 and a storage unit 242. Various kinds of programs and data (including programs and data for calculating blood sugar level data based on the photometric data input via the A/D converter 250) for implementing the functions of the blood sugar measurement device 200 are stored in the storage unit 242 and appropriately executed by the CPU 241.

The storage unit 242 also stores a communication control program 243 used to transmit an identifier corresponding to the internal state of the blood sugar measurement device 200 or calculated blood sugar level data to the portable phone 300. The blood sugar measurement device is thus configured to be able to successively transmit information (an identifier corresponding to the internal state or blood sugar level data) to the portable phone 300 via the communication unit 280 and cause the portable phone 300 to execute predetermined voice output at the time of blood sugar measurement processing.

the display control unit 260 controls to display a predetermined screen (a screen to display calculated blood sugar level data or information representing the internal state of the control unit 240) on the display unit 214 based on a display instruction from the control unit 240. The input acceptance unit 270 accepts an instruction from the power switch 213, the history call button 215, or the reproduction button 216 in Fig. 1 and transmits the accepted instruction to the control unit 240.

At the time of blood sugar measurement processing, the communication unit 280 transmits an identifier corresponding to the internal state of the blood sugar measurement device 200 or calculated blood sugar level data to the portable phone 300. Note that the communication method of the communication unit 280 is not limited to a specific communication method and can use any of a radio wave, light, and a sound wave.

As described above, in this embodiment, the blood sugar measurement device 200 is provided with the communication control program 243 and the communication unit 280 and configured to successively transmit an identifier corresponding to the internal state of the blood sugar measurement device or blood sugar level data as a measurement result to the portable phone 300. This allows the portable phone 300 to perform voice output. As a result, the blood sugar measurement device 200 need not have the voice output function and can have smaller outer dimensions (that is, it is possible to provide a blood sugar measurement device having excellent operability and portability).

### <3. Functional Arrangement of Portable Phone And That of Voice Output Control Program>

The functional arrangement of the portable phone 300 and that of an voice output control program included in the application for the blood sugar measurement device will be described next.

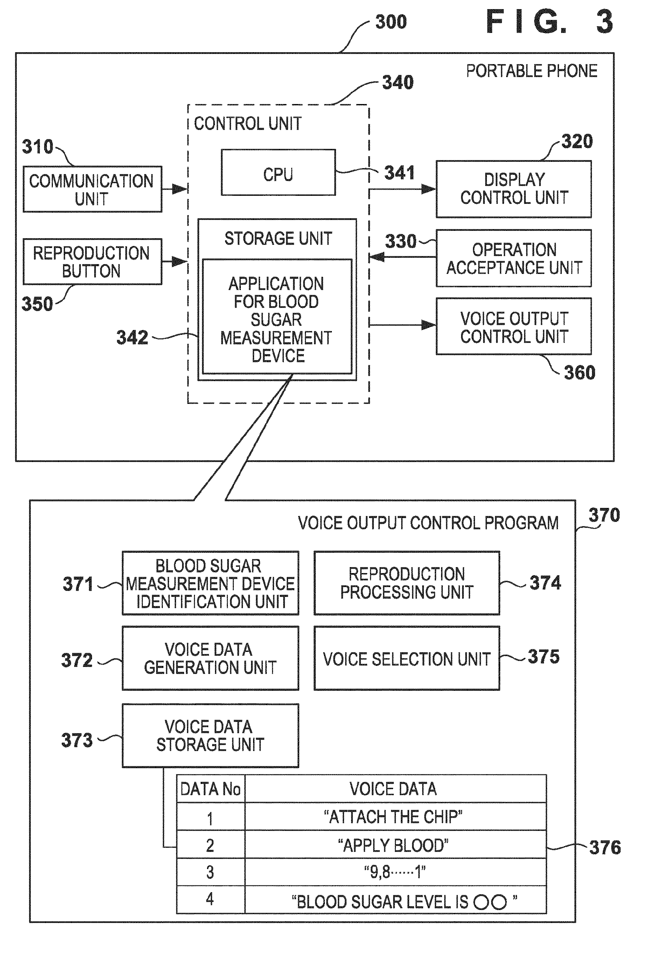

Fig. 3 is a block diagram showing the functional arrangement of the portable phone 300. Note that the portable phone 300 is assumed to have the functions of a general portable phone such as a communication function and a mail sending/receiving function. In the example of Fig. 3, only the functional arrangement concerning execution of the application (especially voice output control processing) for the blood sugar measurement device is illustrated in particular.

As shown in Fig. 3, the portable phone 300 includes a communication unit 310, a control unit 340, a display control unit 320, an operation acceptance unit 330, a voice output control unit 360, and a reproduction button 350.

The communication unit 310 is used when communicating with the blood sugar measurement device 200. Note that the communication with the blood sugar measurement device 200 can be done by an arbitrary communication method using a radio wave, light, a sound wave, or the like, as described above.

The display control unit 320 controls to display, on the display unit 312, a menu screen to select the application for the blood sugar measurement device or the operation screen 315 of the application for the blood sugar measurement device when it is selected.

The operation acceptance unit 330 accepts an operation of the subject from the operation unit 311 including arrow keys to move a cursor to a selected item displayed on the menu screen or the operation screen, an enter key to select a selected item at the position of the moved cursor, and various ten keys.

The voice output control unit 360 controls to output, via the voice output unit 314, voice data selected by the application for the blood sugar measurement device based on an instruction from the blood sugar measurement device 200. Note that the voice output unit 314 is configured to output a voice on the communication partner side during speech communication (that is, voice data output by the application for the blood sugar measurement device is implemented by sharing the voice output unit for outputting the voice on the communication partner side at the time of speech communication).

The reproduction button 350 is used to input an instruction to output the voice data output from the voice output unit 314 based on an instruction from the blood sugar measurement device 200 again. When the reproduction button 350 is pressed, the voice data output most recently is output again. Note that the reproduction button 350 can be provided separately from the operation unit 311, or one of the keys included in the operation unit 311 may be configured to function as the reproduction button 350.

The control unit (computer) 340 includes a CPU 341 and a storage unit 342. The application for the blood sugar measurement device is stored in the storage unit 342 and executed by the CPU 341.

Note that a program (voice output control program 370) to implement processing executed in the voice output mode out of the modes of the application for the blood sugar measurement device includes a blood sugar measurement device identification unit 371, a voice data generation unit 372, a voice data storage unit 373, a reproduction processing unit 374, and a voice selection unit 375.

When the application for the blood sugar measurement device is activated, and the voice output mode is automatically selected to start the operation, the blood sugar measurement device identification unit 371 receives a signal from the blood sugar measurement device 200, thereby identifying the blood sugar measurement device as the communication target.

The voice data storage unit 373 stores voice data output based on an instruction from the blood sugar measurement device 200. The voice data is assumed to be stored in association with an identifier (data No) corresponding to the internal state of the blood sugar measurement device (see 376).

When blood sugar level data is transmitted from the blood sugar measurement device 200, the voice data generation unit 372 synthesizes it with predetermined voice data stored in the voice data storage unit 373, thereby generating voice data to output the measurement result as a voice.

The voice selection unit 375 selects corresponding voice data from voice data stored in the voice data storage unit 373 based on an instruction (including the identifier corresponding to the internal state of the blood sugar measurement device) from the blood sugar measurement device 200, and inputs the voice data to the reproduction processing unit 374. When blood sugar level data is transmitted from the blood sugar measurement device 200, and the voice data generation unit 372 generates voice data to output the measurement result as a voice, the generated voice data is input to the reproduction processing unit 374.

The reproduction processing unit 374 outputs the voice data input from the voice selection unit 375 via the voice output unit 314 as a voice.

### <4. Procedure of Blood Sugar Measurement/Output Processing of Blood Sugar Measurement System>

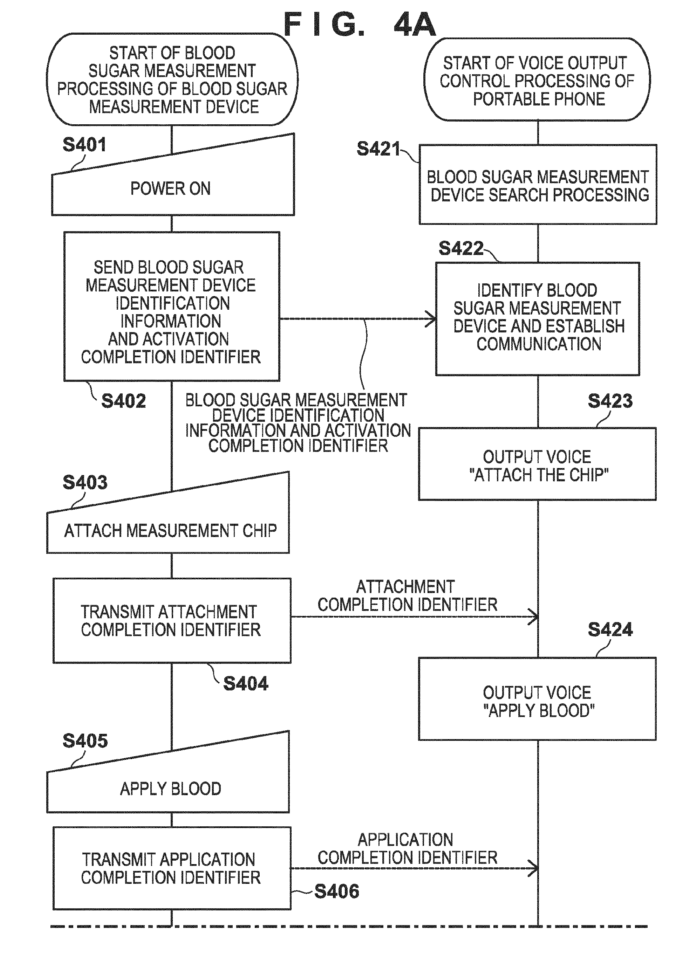

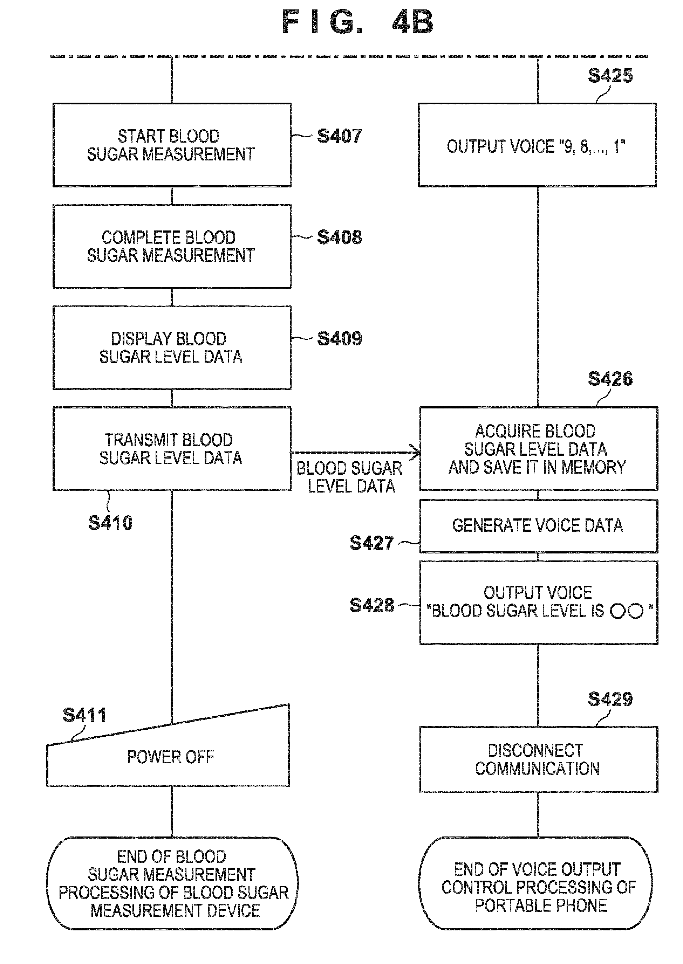

The procedure of blood sugar measurement/output processing of the blood sugar measurement system 100 will be described next. Figs. 4A and 4B are flowcharts showing the procedure of blood sugar measurement/output processing of the blood sugar measurement system 100. As shown in Figs. 4A and 4B, in the blood sugar measurement system 100, the portable phone 300 executes voice output control processing in accordance with execution of blood sugar measurement processing of the blood sugar measurement device 200.

More specifically, when the portable phone 300 is powered on, the application for the blood sugar measurement device is activated, and the voice output mode is automatically selected, the voice output control program 370 is executed, and voice output control processing based on an instruction from the blood sugar measurement device 200 starts.

When the voice output control processing starts, the blood sugar measurement device identification unit 371 executes blood sugar measurement device search processing in step S421, and confirms the presence/absence of signal transmission from the blood sugar measurement device 200.

On the other hand, when powered on in step S401, the blood sugar measurement device 200 outputs, in step S402, a signal including identification information for identifying the blood sugar measurement device 200 and an identifier (data No. 1) representing that the blood sugar measurement device 200 is powered on, and its activation is completed.

In step S422, the portable phone 300 receives the signal sent from the blood sugar measurement device 200, identifies the blood sugar measurement device 200, and establishes communication with the blood sugar measurement device 200. Note that after the establishment of communication with the blood sugar measurement device 200, control is performed to prohibit reception of a signal from any blood sugar measurement device other than the blood sugar measurement device 200 until the communication is disconnected.

When the communication between the portable phone 300 and the blood sugar measurement device 200 is established, the portable phone 300 outputs voice data "attach the chip" corresponding to the received identifier (data No. 1) in step S423.

In accordance with the voice output of the portable phone 300, the subject attaches the measurement chip 220 to the measurement device main body 210 in step S403. When the measurement chip 220 is attached to the measurement device main body 210, the measurement device main body 210 recognizes this and transmits an identifier (data No. 2) representing completion of the attachment to the portable phone 300 in step S404.

Upon receiving the identifier representing completion of the attachment, the portable phone 300 outputs voice data "apply blood" corresponding to the received identifier (data No. 2) in step S424.

In accordance with the voice output of the portable phone 300, the subject applies blood to the measurement chip 220 in step S405. Upon detecting the application to the measurement chip 220, the blood sugar measurement device 200 transmits an identifier (data No. 3) representing completion of the application to the portable phone 300 in step S406, and starts blood sugar measurement in step S407.

Upon receiving the identifier representing completion of the application, the portable phone 300 outputs voice data "9, 8,..., 1" corresponding to the received identifier (data No. 3) in step S425.

Before or at the same time as completion of the countdown voice output of the portable phone 300, the blood sugar measurement device 200 completes blood sugar measurement (step S408).

The blood sugar measurement device 200 that has completed the blood sugar measurement displays blood sugar level data as the measurement result on the display unit 214 in step S409.

In step S410, the blood sugar measurement device 200 transmits the blood sugar level data as the measurement result to the portable phone 300. Upon receiving the blood sugar level data as the measurement result transmitted from the blood sugar measurement device 200, the portable phone 300 saves the received blood sugar level data in the memory in association with the reception date/time in step S426.

In step S427, voice data is generated based on the blood sugar level data as the measurement result received in step S426. More specifically, the blood sugar level data as the measurement result received in step S426 is synthesized with voice data represented by data No. 4, thereby generating voice data "blood sugar level is OO".

In step S428, the voice data generated in step S427 is output. After the voice data is output, the processing waits until the blood sugar measurement device 200 is powered off. When the blood sugar measurement device 200 is powered off in step S411, the portable phone 300 disconnects the communication with the blood sugar measurement device 200 in step S429.

Note that after the end of the communication with the blood sugar measurement device 200, the voice output control processing may be ended, or the process may return to step S421 to search for another blood sugar measurement device.

### <5. Procedure of Reproduction Processing of Portable Phone>

The procedure of reproduction processing of the portable phone 300 will be explained next. The portable phone 300 is configured to perform voice data reproduction processing when the reproduction button 216 of the blood sugar measurement device 200 is pressed or when the reproduction button 350 of the portable phone 300 is pressed.

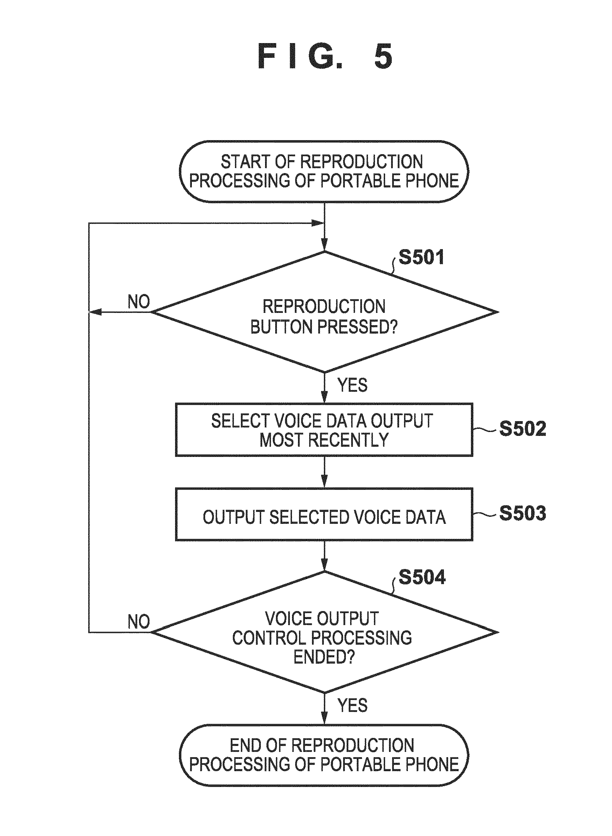

More specifically, as shown in Fig. 5, reproduction processing starts at the same time as the start of voice output control processing, and the portable phone 300 starts determining whether a signal representing that the reproduction button 216 is pressed is received from the blood sugar measurement device 200 or the reproduction button 350 of the portable phone 300 is pressed (step S501).

Upon determining in step S501 that a signal representing that the reproduction button 216 is pressed is received or determining that the reproduction button 350 of the portable phone 300 is pressed, the process advances to step S502.

In step S502, the voice selection unit 375 identifies voice data selected most recently and inputs the voice data to the reproduction processing unit 374. Note that when the voice data selected most recently is voice data for outputting blood sugar level data as a measurement result as a voice, the generated voice data is input to the reproduction processing unit 374.

In step S503, control is performed such that the reproduction processing unit 374 outputs the input voice data via the voice output unit 314 as a voice.

In step S504, it is determined whether the voice output control processing has ended. If the voice output control processing has not ended yet, the process returns to step S501. Upon determining that the voice output control processing has ended, the reproduction processing also ends.

The reproduction processing is executed in the above-described way. Hence, for example, when the reproduction button is pressed after step S423 of Fig. 4A, the voice data output as a voice in step S423 is output again. When the reproduction button is pressed after step S424, the voice data output as a voice in step S424 is output again.

When the reproduction button is pressed after step S428, the voice data output as a voice in step S428 is output again.

That is, when one of the reproduction buttons arranged on the blood sugar measurement device and the portable phone is pressed, the voice data most recently output as a voice is reproduced. Even if the subject has missed the voice data, he/she can hear it again.

As is apparent from the above description, the portable phone 300 according to this embodiment is configured to store voice data representing the operation contents of the blood sugar measurement device 200 and voice data used to output blood sugar level data that is a measurement result as a voice, and upon receiving, from the blood sugar measurement device 200, an identifier corresponding to its internal state, output voice data representing the corresponding operation contents. The portable phone 300 is also configured to, upon receiving blood sugar level data as a measurement result, generate voice data used to output the blood sugar level data as a voice by synthesizing the blood sugar level data with the voice data used to output the blood sugar level data as a voice and output the voice data.

This allows the portable phone to output operation guidance corresponding to the internal state of the blood sugar measurement device at the time of measurement or blood sugar level data as a measurement result as a voice and makes it possible to implement the voice output function without imparting the voice output function to the blood sugar measurement device.

That is, it is possible to implement the voice output function while maintaining the operability and portability of the blood sugar measurement device.

### [Second Embodiment]

In the first embodiment, voice output control processing of the portable phone 300 when blood sugar measurement processing is performed in the measurement mode of the blood sugar measurement device 200 has been described. However, the present invention is not limited to this. For example, the present invention is also applicable when calling past blood sugar level data measured in the past by a blood sugar measurement device 200 and stored in a storage unit 242 in association with the measurement date/time (that is, when a history call button 215 is pressed to transit to the history call mode).

In this embodiment, voice output control processing of a portable phone 300 when the history call button 215 of the blood sugar measurement device 200 is pressed will be described.

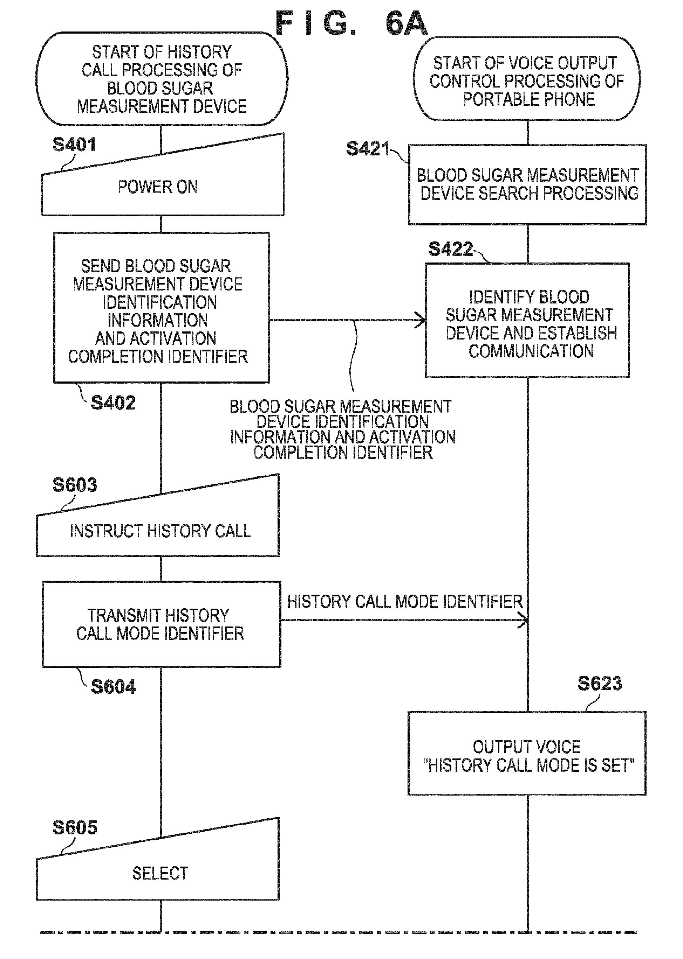

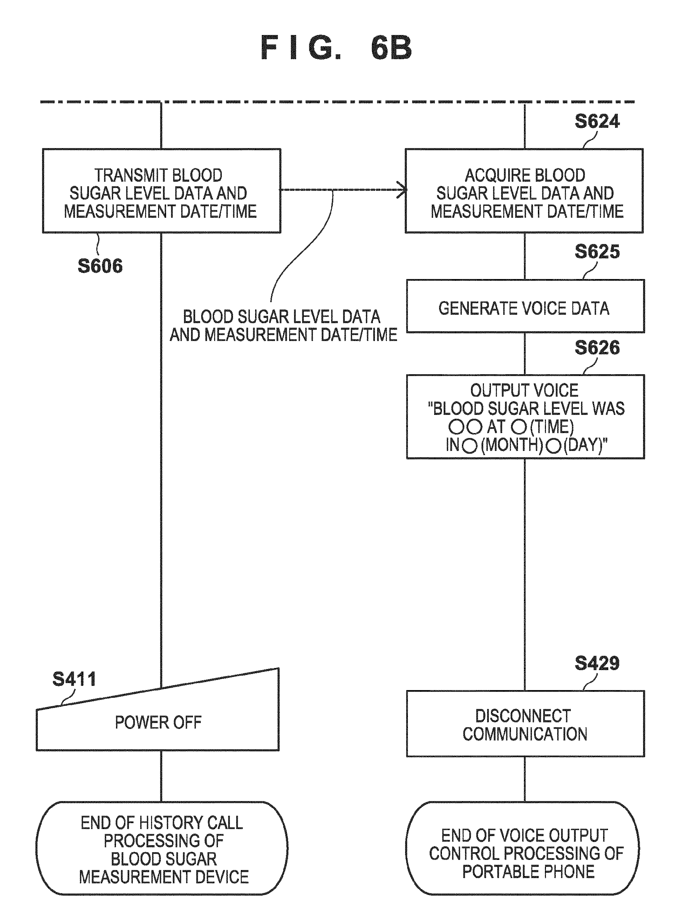

Figs. 6A and 6B are flowcharts showing the procedure of history call/output processing of a blood sugar measurement system 100. Note that in Fig. 6A, processing after the portable phone 300 and the blood sugar measurement device 200 are powered on until communication between them is established is the same as in the first embodiment, and a description thereof will be omitted here.

When the subject presses the history call button 215 of the blood sugar measurement device 200 that has established communication with the portable phone 300 (step S603), an identifier representing that the blood sugar measurement device 200 has shifted to the history call mode is transmitted to the portable phone 300 in step S604.

Upon receiving the identifier representing the shift to the history call mode, the portable phone 300 outputs voice data "history call mode is set" corresponding to the identifier in step S623. Note that the voice data is assumed to be stored in a voice data storage unit 373 in advance in association with a predetermined data No.

The blood sugar measurement device 200 that has shifted to the history call mode calls past blood sugar level data stored in the storage unit 242 in association with the measurement date/time and displays it on a display unit 214. More specifically, when the history call button 215 is pressed once, blood sugar level data measured last time and its measurement date/time are displayed. When the history call button 215 is pressed once more, blood sugar level data measured second previous time and its measurement date/time are displayed. When the history call button 215 is pressed long, the past blood sugar level data and their measurement dates/times are fast-forwarded.

When the subject selects past blood sugar level data by pressing the history call button 215 a desired number of times in step S605, the blood sugar level data displayed on the display unit 214 after the press and information about the measurement date/time at which the blood sugar level data was measured are transmitted to the portable phone 300 in step S606.

When the blood sugar level data and the information about the measurement date/time are transmitted from the blood sugar measurement device 200, the portable phone 300 receives them (step S624). In step S625, voice data is generated based on the blood sugar level data and the measurement date/time received in step S624. More specifically, voice data "blood sugar level was OO at O (time) in O (month) O (day)" is generated. Note that the voice data storage unit 373 stores in advance voice data used to output blood sugar level data and the measurement date/time of the blood sugar level data as a voice.

In step S626, the voice data generated in step S625 is output. After the voice data is output, the processing waits until the blood sugar measurement device 200 is powered off. When the blood sugar measurement device 200 is powered off in step S410, the portable phone 300 disconnects the communication with the blood sugar measurement device 200 in step S429.

As is apparent from the above description, the portable phone 300 according to this embodiment is configured to, when the blood sugar measurement device 200 performs history call processing and selects predetermined blood sugar level data, receive the selected blood sugar level data and the measurement date/time information of the blood sugar level data, generate voice data, and output the voice data.

This allows to cope with a case in which the history call button of the blood sugar measurement device 200 is pressed.

Note that a case has been described above in which the blood sugar measurement device 200 shifts to the history call mode when the history call button is pressed after powered on. However, the present invention is not limited to this and is also applicable to a case in which the blood sugar measurement device 200 shifts to the history call mode when the history call button is pressed after blood sugar measurement processing.

In the above description, a case in which the reproduction button is pressed after the shift to the history call mode has not particularly been mentioned. Reproduction processing of the portable phone 300 when the reproduction button is pressed after the shift to the history call mode is basically the same as the reproduction processing described with reference to Fig. 5. That is, upon determining that a signal representing that the reproduction button is pressed is received, voice data (voice data including blood sugar level data and its measurement date/time) output most recently is output again.

In the above description, when the history call button 215 is pressed, the blood sugar measurement device immediately shifts to the history call mode. However, the present invention is not limited to this. The portable phone may be configured to prohibit reception of the history call mode identifier from the blood sugar measurement device 200 during the time after the application completion identifier is transmitted (step S406) until blood sugar level data is acquired (step S426), in which the blood sugar measurement device 200 is in the measurement mode.

In the above description, the blood sugar measurement device is configured to select past blood sugar level data in accordance with the number of times of pressing the history call button 215. However, the present invention is not limited to this. For example, the blood sugar measurement device may be configured to transmit blood sugar level data and information about the measurement date/time to the portable phone 300 using, as a trigger, press of another button (for example, reproduction button 216) in a state in which past blood sugar level data is displayed on the display unit 214 in accordance with the number of times of pressing the history call button 215.

In the above description, the portable phone is configured to immediately output voice data upon receiving blood sugar level data and information about the measurement date/time. However, the present invention is not limited to this. For example, the portable phone may be configured to output voice data when the reproduction button of the portable phone 300 is pressed.

### [Other Embodiments]

In the first and second embodiments, the blood sugar measurement device 200 having the voice output function has been exemplified. However, the present invention is not limited to this, and any other medical device is usable as long as it has the voice output function.

The present invention is not limited to the above embodiments and various changes and modifications can be made within the spirit and scope of the present invention. Therefore, to apprise the public of the scope of the present invention, the following claims are made.

This application claims the benefit of Japanese Patent Application No. 2010-293748, filed December 28, 2010, which is hereby incorporated by reference herein in its entirety.

## Claims

1. A portable communication device including an application for implementing a voice output function, **characterized by** comprising:
communication means for starting communication to receive a signal transmitted from a predetermined medical device by activating the application;
storage means for storing voice data representing operation contents of the predetermined medical device;
selection means for, when a signal including an identifier corresponding to an internal state of the predetermined medical device is received from the predetermined medical device, selecting the voice data according to the internal state out of the voice data stored in said storage means; and
output means for outputting the voice data selected by said selection means.

2. The portable communication device according to claim 1, **characterized in that**
said storage means stores the voice data in association with the identifier corresponding to the internal state of the predetermined medical device, and
said selection means selects the voice data stored in association with the identifier included in the received signal and corresponding to the internal state of the predetermined medical device.

3. The portable communication device according to claim 2, **characterized in that**
said storage means further stores voice data used to output measurement data measured by the predetermined medical device as a voice,
the portable communication device further comprises generation means for, upon receiving the measurement data measured by the predetermined medical device, generating the voice data used to output the measurement data as the voice by synthesizing the voice data stored in said storage means with the received measurement data, and
when the measurement data measured by the predetermined medical device is received from the predetermined medical device, said selection means selects the voice data generated by said generation means.

4. The portable communication device according to claim 3, **characterized in that**
said storage means further stores voice data used to output the measurement data measured by the predetermined medical device and information about a measurement date/time of the measurement data as the voice,
upon receiving the measurement data measured by the predetermined medical device and the information about the measurement date/time of the measurement data, said generation means generates the voice data used to output the measurement data as the voice by synthesizing the voice data stored in said storage means with the received measurement data and the information about the measurement date/time, and
when the measurement data measured by the predetermined medical device and the information about the measurement date/time are received from the predetermined medical device, said selection means selects the voice data generated by said generation means.

5. The portable communication device according to claim 3 or 4, **characterized by** further comprising instruction means for inputting an instruction to reproduce the voice data output by said output means,
wherein when a reproduction instruction is input via said instruction means, said selection means selects voice data selected most recently, and said output means outputs the voice data selected by said selection means.

6. The portable communication device according to claim 3 or 4, **characterized in that** when an instruction to reproduce the voice data is transmitted from the predetermined medical device, said selection means selects voice data selected most recently, and said output means outputs the voice data selected by said selection means.

7. A voice output system **characterized by** comprising a portable communication device of any one of claims 1 to 6, and a predetermined medical device.

8. A method of controlling a portable communication device that includes an application for implementing a voice output function, and stores voice data representing operation contents of a predetermined medical device in a storage unit, **characterized by** comprising:
a communication step of starting communication to receive a signal transmitted from the predetermined medical device by activating the application;
a selection step of, when a signal including an identifier corresponding to an internal state of the predetermined medical device is received from the predetermined medical device, selecting the voice data according to the internal state out of the voice data stored in the storage unit; and
a voice output step of outputting the voice data selected in the selection step as a voice.

9. A program for implementing a voice output function, the program causing a computer of a portable communication device that stores voice data representing operation contents of a predetermined medical device in a storage unit to execute:
a communication step of starting communication to receive a signal transmitted from the predetermined medical device by activating the program;
a selection step of, when a signal including an identifier corresponding to an internal state of the predetermined medical device is received from the predetermined medical device, selecting the voice data according to the internal state out of the voice data stored in the storage unit; and
a voice output step of outputting the voice data selected in the selection step as a voice.
